# EUROPEAN PATENT SPECIFICATION

(11) **EP 3 690 513 B1**
(45) Date of publication and mention of the grant of the patent: **07.06.2023**
(21) Application number: 18860332.8
(22) Date of filing: 28.08.2018
(51) Int. Cl.: G02B 21/36, C12M 1/34, G01N 21/17, G02B 21/26, G02B 21/34, G06T 7/33, G02B 21/14, G02B 21/24

(54) **OBSERVATION DEVICE, OBSERVATION METHOD, AND OBSERVATION PROGRAM**
BEOBACHTUNGSVORRICHTUNG, BEOBACHTUNGSVERFAHREN UND BEOBACHTUNGSPROGRAMM
DISPOSITIF D'OBSERVATION, PROCÉDÉ D'OBSERVATION ET PROGRAMME INFORMATIQUE

(30) Priority: 27.09.2017 JP 2017186414
(43) Date of publication of application: 05.08.2020
(73) Proprietor: FUJIFILM Corporation, Tokyo 106-8620 (JP)
(72) Inventor: MATSUBARA Kenta, Kanagawa 258-8538 (JP)
(74) Representative: Klunker IP Patentanwälte PartG mbB
(86) International application number: PCT/JP2018/031777
(87) International publication number: WO 2019/065050

(56) References cited:
- EP-A1- 1 377 865
- WO-A1-2013/105373
- JP-A- 2011 141 391
- JP-A- 2016 125 913
- US-A1- 2002 154 216
- US-A1- 2009 317 895
- US-A1- 2012 307 047
- US-A1- 2013 162 803

## Description

### BACKGROUND OF THE INVENTION

### 1. Field of the Invention

The technology of the disclosure relates to an observation apparatus, an observation method, and an observation program for observing an observation target by relatively moving at least one of a container accommodating the observation target or an imaging unit with respect to the other.

### 2. Description of the Related Art

In recent years, various technologies for imaging a subject such as various cells and analyzing an acquired image have been suggested. For example, a method of imaging a pluripotent stem cell such as an embryonic stem (ES) cell and an induced pluripotent stem (iPS) cell, a differentiation-induced cell, or the like using a microscope or the like and determining a differentiation state or the like of the cell by recognizing a feature of the image has been suggested.

The pluripotent stem cell such as the ES cell and the iPS cell has a capability to differentiate into cells of various tissues and has drawn attention for its applicability in regenerative medicine, development of medication, identification of diseases, and the like.

In a case where the cell is imaged as described above, it is preferable to acquire a high magnification wide field-of-view image. In order to do so, for example, JP2016-071117A suggests an imaging method of scanning a range of a cultivation container such as a well plate or a dish by an image forming optical system, capturing an image at a series of predetermined imaging positions, and then, combining a partial image obtained at each imaging position. Consequently, by combining the partial images, the entire cell is imaged in a wide field of view. Such an imaging method is referred to as tiling imaging, and each partial image is referred to as a tile image.

In a case where the image forming optical system relatively scans the cultivation container, a positional deviation occurs due to a change in speed, vibration, or the like, and it is difficult to output accurate partial images.

Therefore, JP2016-125913A discloses a technology for performing re-imaging in a case where a defect occurs in a part of the tile images in the tiling imaging.

US 2012/307047 A1 discloses an observation apparatus for obtaining an image of a specimen captured by an image sensor which has a limited field of view so that it can capture only a part of the entire specimen. To this end, a whole image area is subdivided by imaginary dividing lines. Such dividing lines are arranged at arbitrary positions within a reference block. The reference block may be moved in such a way that a minimum number of complete blocks covers the whole area of the reference block. Having captured partial images corresponding to the blocks, such blocks are stitched together to obtain an entire image.

### SUMMARY OF THE INVENTION

However, in the technology disclosed in JP2016-125913A, imaging has to be performed again each time a defect occurs in a part of the tile images. Thus, a processing time for obtaining the final entire image of the cell or the like is increased as the re-imaging is performed.

The technology of the disclosure is conceived in view of the above point, and an object of the technology is to provide an observation apparatus, an observation method, and an observation program capable of acquiring consistent partial images without performing re-imaging.

The predetermined imaging position is not a position of the imaging unit in a focus direction (Z direction) and is a position in a plane (XY plane) in which the container is placed. The partial image is an image that is obtained by imaging by the imaging unit in a field of view of the imaging unit at each of a plurality of predetermined imaging positions. The partial shape is a part that represents a feature of a shape of the accommodation part of the container included in an imaging field of view of the partial image. For example, the partial shape is a shape of a part of an edge of a well in a case where the accommodation part is a well constituting a recessed portion. The partial shape information is shape information representing the partial shape of the container included in the partial image and is information indicating the shape and a position of the accommodation part included in the partial image. The reference shape information of the container is information that is acquired and stored inthe storage unit in advance before capturing of the partial image, and is information representing the shape (reference shape) of the container as a reference such as the shape of the accommodation part and the position of the accommodation part in the entire container. Consistency means matching a position of the partial shape of the container in a boundary part between adjacent partial images or reducing a positional deviation in a case where matching cannot be performed.

In the observation apparatus, the calculation unit may calculate reference partial shape information corresponding to an imaging field of view of each partial image from the reference shape information based on the predetermined imaging positions of the series of partial images, and based on the partial shape information in one partial image of the adjacent partial images and the reference partial shape information corresponding to the imaging field of view of the other partial image scheduled to be captured subsequently to the one partial image, the control unit may cause the other partial image to be consistent with the one partial image by correcting the imaging position of the other partial image from the predetermined imaging positions. The reference partial shape information is information that indicates the reference shape (reference partial shape) of the container in each imaging field of view in which the reference shape of the entire container indicated by the reference shape information is divided in units of imaging field of views of the partial images of the imaging unit at each of the predetermined imaging positions.

In the observation apparatus, the control unit may reduce a deviation between a position of a partial shape of the accommodation part included in the one partial image and a position of a partial shape of the accommodation part included in the other partial image in a boundary part between the adjacent partial images by correcting the imaging position of the other partial image from the predetermined imaging positions. The boundary part includes an adjacent side of the adjacent partial images. For example, the boundary part may be a region that extends inwards at a predetermined ratio from the adjacent side, or may be boundary lines of the adjacent partial images.

In the observation apparatus, the storage unit may store shape information representing a shape of an edge of the accommodation part included in the container as the reference shape information, and the calculation unit may calculate shape information representing a shape of an edge of the accommodation part included in the partial image as the partial shape information.

The observation apparatus may further comprise an input unit into which the referenceshape information is input, in which the storage unit stores the reference shape information input into the input unit.

The observation apparatus may further comprise a measurement unit that acquires the reference shape information by measuring the shape of the container, in which the storage unit stores the reference shape information obtained by measurement performed by the measurement unit.

An observation method according to the technology of the disclosure comprises the features of claim 7.

A non-transitory computer-readable recording medium according to the technology of the disclosure comprises the features of claim 8.

Another observation apparatus according to the technology of the disclosure comprises a memory that stores an instruction to be executed by a computer, and a processor configured to execute the stored instruction, in which the processor executes an imaging step of imaging a container accommodating an observation target in an accommodation part and the observation target in a field of view smaller than the accommodation part at predetermined imaging positions and acquiring a series of partial images, a calculation step of calculating partial shape information representing a partial shape of the container from the partial image obtained by imaging performed in the imaging step, a storage step of storing reference shape information representing a shape of the container, and a control step of correcting the imaging position in a case where the partial image scheduled to be subsequently captured during acquisition of the series of partial images is captured based on the partial shape information and the reference shape information, and causing the series of partial images in which adjacent partial images are consistent to be captured in the imaging step based on the corrected imaging position.

According to the technology of the disclosure, the control unit corrects the imaging position in a case where the partial image scheduled to be subsequently captured is captured based on the reference shape information of the container stored in the storage unit and the partial shape information calculated by the calculation unit, and causes the series of partial images in which the adjacent partial images are consistent to be captured based on the corrected imaging position. Since the imaging position of the imaging unit is appropriately corrected during acquisition of the series of partial images and the movement unit and the imaging unit are controlled, re-imaging after the end of a series of imaging or re-imaging of the partial images at the same position by suspending the series of imaging does not occur. Consequently, re-imaging is not necessary, and consistent adjacent partial images can be acquired.

### BRIEF DESCRIPTION OF THE DRAWINGS

Fig. 1 is a diagram illustrating a schematic configuration of an observation apparatus according to an embodiment of the technology of the disclosure.
Fig. 2 is a diagram illustrating one example of a placing stand.
Fig. 3 is a block diagram illustrating a configuration of a control unit according to the embodiment of the technology of the disclosure.
Fig. 4 is a diagram illustrating a scanning path by a solid line M in a cultivation container.
Fig. 5 is a diagram illustrating a positional relationship among a first displacement sensor, a second displacement sensor, and the cultivation container in a case where a field of view is present at any position in the cultivation container.
Fig. 6 is a diagram illustrating a positional relationship among the first displacement sensor, the second displacement sensor, and the cultivation container in a case where the field of view is present at any position in the cultivation container.
Fig. 7 is a diagram illustrating an example of partial images acquired by scanning of a microscope device.
Fig. 8 is a flowchart illustrating a flow of observation method executed by the observation apparatus.
Fig. 9 is a flowchart illustrating a detailed flow of capturing of the partial images.
Fig. 10 is a diagram illustrating a deviation between a partial shape and a reference partial shape.
Fig. 11 is a diagram illustrating a timing of operation performed by the control unit.

### DESCRIPTION OF THE PREFERRED EMBODIMENTS

Hereinafter, one example of an embodiment according to the technology of the disclosure will be described with reference to the drawings. The same or equivalent constituents and parts in each drawing will be designated by the same reference signs. Dimensional ratios in the drawings are exaggerated for convenience of description and may be different from the actual ratios.

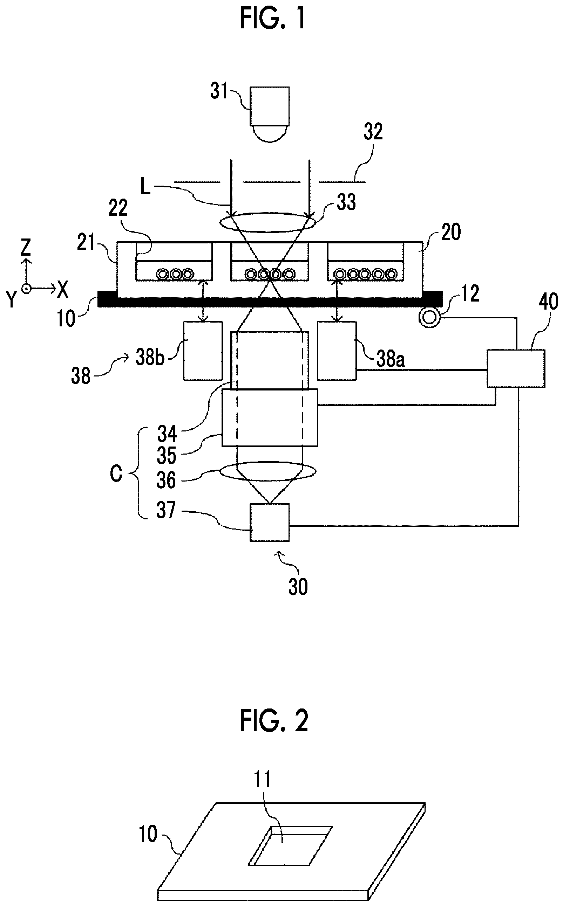

Fig. 1 is a diagram illustrating a schematic configuration of an observation apparatus according to the embodiment of the technology of the disclosure. Fig. 2 is a diagram illustrating one example of a placing stand.

The observation apparatus is an apparatus for observing an observation target accommodated in a cultivation container 20 placed on a placing stand 10 by a microscope device 30. The placing stand 10 and the microscope device 30 are controlled by a control unit 40. Each configuration will be described in order.

The placing stand 10 is a stage on which the cultivation container 20 can be placed. As illustrated in Fig. 2, a rectangular opening 11 is formed at the center of the placing stand 10. It is configured that the cultivation container 20 is installed on a member forming the opening 11, and light for observation by the microscope device 30 passes through the cultivation container 20.

A movement unit 12 is attached to the placing stand 10. The movement unit 12 can freely move the placing stand 10 in an X direction and a Y direction that are orthogonal to each other. The X direction and the Y direction are directions orthogonal to a Z direction and are directions orthogonal to each other in a horizontal plane. In the present embodiment, the X direction is set as a main scanning direction, and the Y direction is set as a sub-scanning direction. The movement unit 12 is configured with an actuator that includes a piezoelectric element or the like. Movement of the placing stand 10 in an XY plane is controlled by the control unit 40. By moving the placing stand 10 in the XY plane, the cultivation container 20 on the placing stand 10 moves with respect to the microscope device 30.

In the present embodiment, an example in which a position at which the observation target is observed by the microscope device 30 is changed by moving the placing stand 10 with respect to the microscope device 30 is illustrated. However, the example is not for limitation purposes. The microscope device 30 may be moved with respect to the placing stand 10, or both of the placing stand 10 and the microscope device 30 may be moved. Any aspect can be employed as long as at least one of the cultivation container 20 placed on the placing stand 10 or the microscope device 30 is relatively moved with respect to the other. In the present disclosure, for example, the microscope device 30 is represented as "relatively moving" with respect to the cultivation container 20 even in a case where a position of the microscope device 30 is fixed and only the cultivation container 20 is moving.

Instead of placing the cultivation container 20 on the placing stand 10 and moving the cultivation container 20, the cultivation container 20 may be moved in the X-Y plane by moving a holding unit that holds at least a part of the cultivation container 20.

In the cultivation container 20, a plurality of accommodation parts 22 are formed in a plate 21 having a flat plate shape. For example, a Petri dish, a dish, or a well plate can be used as the cultivation container 20. For example, the accommodation part 22 is a recessed portion having a circular shape in a plan view and is referred to as a well. The accommodation part 22 accommodates the observation target such as various cells immersed in a cultivation liquid. Cells accommodated in the accommodation part 22 include pluripotent stem cells such as an iPS cell and an ES cell, cells of a nerve, skin, cardiac muscle, and a liver that are differentiation-induced from a stem cell, cells of skin, a retina, cardiac muscle, a blood cell, a nerve, and an organ extracted from a human body, and the like.

The microscope device 30 captures a phase difference image of the observation target. In order to obtain a high magnification image, the microscope device 30 captures partial images of the observation target and the cultivation container 20 in a field of view smaller than each accommodation part 22 of the cultivation container 20. As described above, by moving the cultivation container 20 with respect to the microscope device 30, the microscope device 30 scans the cultivation container 20, and a series of partial images is obtained. The partial image is an image that is obtained by imaging by the microscope device 30 in a field of view of the microscope device 30 at each of a plurality of predetermined imaging positions. The predetermined imaging position is not a position of an imaging apparatus in a focus direction (Z direction) and is a position in a plane (XY plane) in which the cultivation container 20 is placed. The position in the focus direction (Z direction) is appropriately adjusted by a focus adjustment mechanism 35 based on a detection result of an autofocus detection unit 38 described later.

The microscope device 30 comprises a light source 31, a slit 32, a condenser lens 33, an objective lens 34, the focus adjustment mechanism 35, an image forming lens 36, an imaging unit 37, and the autofocus detection unit 38.

The light source 31 emits white light. The slit 32 is formed by disposing a ring shaped slit through which the white light is transmitted in a light screen that blocks the white light emitted from the light source 31. Illumination light L having a ring shape is formed by causing the white light to pass through the slit. The condenser lens 33 condenses the illumination light L having the ring shape on the observation target.

The objective lens 34 is arranged to face the condenser lens 33 through the cultivation container 20. The objective lens 34 forms an image of the observation target in the cultivation container 20. The focus adjustment mechanism 35 includes a phase difference lens that can be moved in an optical axis direction (Z direction). By moving the phase difference lens in the optical axis direction, autofocus control is performed, and contrast of the phase difference image captured by the imaging unit 37 is adjusted. The movement of the phase difference lens in the optical axis direction can be implemented by driving an actuator such as a piezoelectric element based on a signal from the control unit 40. However, the piezoelectric element is not for limitation purposes, and the phase difference lens can be driven using other known configurations as long as the phase difference lens can be moved in the Z direction. In addition, a magnification of the phase difference lens may be configured to be changeable. Specifically, a phase difference lens or the focus adjustment mechanism 35 having a different magnification may be configured to be replaceable. The replacement may be automatically performed or may be manually performed by a user.

The phase difference image that passes through the focus adjustment mechanism 35 is incident on the image forming lens 36, and the image forming lens 36 forms the phase difference image on the imaging unit 37.

The imaging unit 37 captures the phase difference image formed by the image forming lens 36. The imaging unit 37 is an imaging element such as a charge-coupled device (CCD) image sensor or a complementary metal-oxide semiconductor (CMOS) image sensor. As the imaging element, an imaging element in which color filters of red, green, blue (RGB) are disposed may be used, or a monochrome imaging element may be used.

Hereinafter, the objective lens 34, the focus adjustment mechanism 35, the image forming lens 36, and the imaging unit 37 will be collectively referred to as an image forming optical system C.

The autofocus detection unit 38 detects a Z-directional position of the cultivation container 20 installed on the placing stand 10. Specifically, the autofocus detection unit 38 comprises a first displacement sensor 38a and a second displacement sensor 38b. The first displacement sensor 38a and the second displacement sensor 38b are arranged in the X direction illustrated in Fig. 1 with the image forming optical system C interposed therebetween. The first displacement sensor 38a and the second displacement sensor 38b in the present embodiment are laser displacement meters and detect a Z-directional position of a bottom surface of the cultivation container 20 by irradiating the cultivation container 20 with laser light and detecting reflected light. The bottom surface of the cultivation container 20 is a boundary surface between a bottom portion of the cultivation container 20 and the cell which is the observation target, that is, an observation target installation surface.

Z-directional positional information of the cultivation container 20 detected by the autofocus detection unit 38 is output to the control unit 40. The control unit 40 performs the autofocus control by controlling the focus adjustment mechanism 35 based on the input positional information. The detection of the position of the cultivation container 20 by the first displacement sensor 38a and the second displacement sensor 38b, and the autofocus control will be described in detail later.

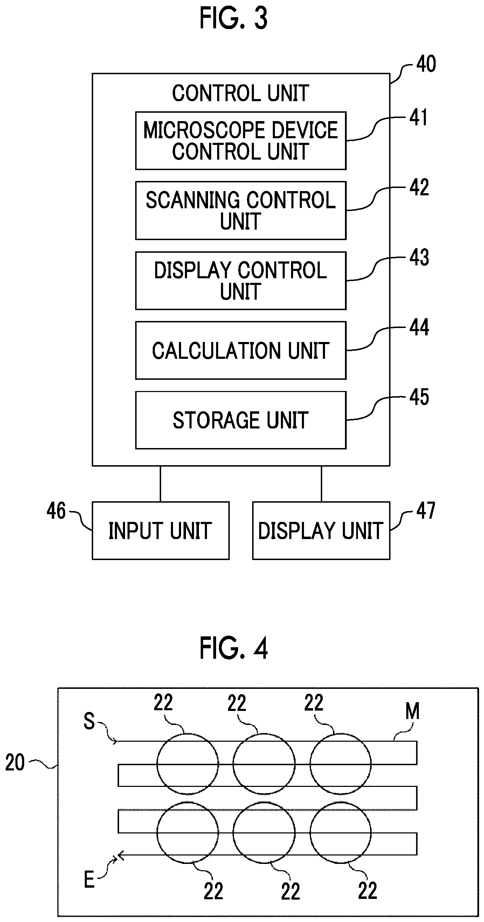

Next, a configuration of the control unit 40 controlling the microscope device 30 will be described. Fig. 3 is a block diagram illustrating a configuration of the control unit according to the embodiment of the technology of the disclosure.

The control unit 40 controls the entire microscope device 30 as described above and executes various processes. The control unit 40 includes a microscope device control unit 41, a scanning control unit 42, a display control unit 43, a calculation unit 44, a storage unit 45, an input unit 46, and a display unit 47. The control unit 40 is configured with a computer that comprises a central processing unit (CPU), a semiconductor memory, and the like. In the control unit 40, an observation program according to one embodiment of the present invention is installed in the storage unit 45. The microscope device control unit 41, the scanning control unit 42, and the display control unit 43 illustrated in Fig. 3 function by causing the CPU to execute the observation program.

The microscope device control unit 41 controls the focus adjustment mechanism 35 based on the Z-directional positional information of the cultivation container 20 detected by the autofocus detection unit 38 as described above. By driving the focus adjustment mechanism 35, the phase difference lens moves in the optical axis direction, and the autofocus control is performed.

In addition, the microscope device control unit 41 controls imaging performed by the imaging unit 37 in a case where the cultivation container 20 is scanned. Specifically, a timing of imaging during scanning is stored in advance in the storage unit 45. The microscope device control unit 41 performs imaging in accordance with the timing.

The scanning control unit 42 controls driving of the movement unit 12 and moves the placing stand 10 in the X direction and the Y direction.

The display control unit 43 generates one composite image by combining the series of partial images captured by the microscope device 30 and displays the composite image on the display unit 47.

The calculation unit 44 calculates partial shape information that is shape information of a partial shape of the cultivation container 20 from the partial images of the cultivation container 20 and the observation target obtained by imaging performed by the imaging unit 37. The partial shape is a part that represents a feature of a shape of the accommodation part 22 of the cultivation container 20 included in an imaging field of view of the partial image. In the present embodiment, the partial shape is a part that represents a feature of the shape and a position of the accommodation part 22 included in the imaging field of view of each partial image. The partial shape information is information that indicates the shape or the position of the accommodation part 22 included in the partial image. In the present embodiment, particularly, the calculation unit 44 calculates the partial shape information from a shape of an edge of the accommodation part 22 of the cultivation container 20 included in the partial image.

The storage unit 45 stores the observation program that implements each function unit. In addition, the storage unit 45 stores, in advance, reference shape information that is obtained by measuring a shape of the cultivation container 20 in advance. The reference shape information is information that is acquired in advance before capturing the partial images, and is obtained by measuring the cultivation container 20 not accommodating the observation target in the accommodation part 22 by a measurement unit such as a laser length measurement device. For example, the reference shape information is information that represents the shape of the accommodation part 22 and the position of the accommodation part 22 in the entire cultivation container 20. In the present embodiment, the reference shape information is the shape of the edge of the accommodation part 22 of the cultivation container 20. The measurement unit used for measurement is preferably a device that has different accuracy from the microscope device 30. Particularly, the measurement unit is preferably a device that can measure the shape of the edge of the accommodation part 22 with higher accuracy than the microscope device 30. The shape of the cultivation container 20 may be measured in advance using the microscope device 30 as the measurement unit.

The reference shape information of the cultivation container 20 stored in the storage unit 45 includes reference partial shape information. The reference partial shape information is information that indicates a reference shape (reference partial shape) of the cultivation container 20 in each imaging field of view in which a reference shape of the entire cultivation container 20 indicated by the reference shape information is divided in units of imaging field of views of the imaging unit 37 at the predetermined imaging positions. Specifically, assuming that the imaging unit 37 reaches the series of imaging positions by scanning, the shape of the edge of the accommodation part 22 of the cultivation container 20 that enters each imaging field of view of the imaging unit 37 is the reference partial shape, and shape information of the reference partial shape is the reference partial shape information. A series of reference partial shape information is obtained in correspondence with all imaging positions. The reference partial shape information may not be stored in the storage unit 45 and may be calculated by the control unit 40 as necessary based on the reference shape information.

The storage unit 45 stores the series of imaging positions of the imaging unit 37 as relative coordinate positions between the placing stand 10 and the microscope device 30.

The input unit 46 comprises a mouse, a keyboard, and the like and receives various necessary data and various setting inputs from the user. For example, the input unit 46 of the present embodiment receives an input of information related to container information of the cultivation container 20, the reference shape information, the reference partial shape information, and the imaging positions. The imaging positions may be determined in advance by the control unit 40 based on the reference shape information input into the input unit 46.

The display unit 47 comprises, for example, a liquid crystal display and displays a composite phase difference image generated by the display control unit 43 as described above. The display unit 47 may be configured with a touch panel and double as the input unit 46.

Next, movement control of the placing stand 10 by the scanning control unit 42 and control of the microscope device 30 by the microscope device control unit 41 will be described in detail.

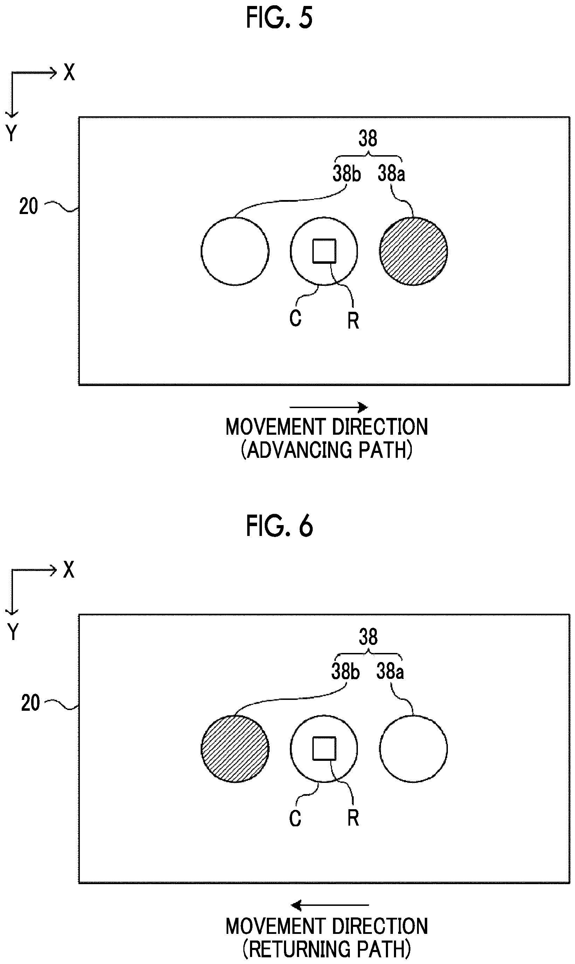

Fig. 4 is a diagram illustrating a scanning path by a solid line M in the cultivation container. Fig. 5 and Fig. 6 are diagrams illustrating a positional relationship among the first displacement sensor, the second displacement sensor, and the cultivation container in a case where the field of view is present at any position in the cultivation container.

In the present embodiment, the placing stand 10 is moved in the X direction and the Y direction under control of the scanning control unit 42, and the microscope device 30 two-dimensionally scans the inside of the cultivation container 20. During the scanning, partial images of the cultivation container 20 and the observation target are captured in each field of view of the microscope device 30. In the present embodiment, a well plate that includes six accommodation parts 22 is used as the cultivation container 20.

As illustrated in Fig. 4, the field of view of the microscope device 30 moves along the solid line M from a scanning start point S to a scanning end point E. That is, the field of view is scanned in a positive direction (a rightward direction in Fig. 4) of the X direction and then, moves in the Y direction (a downward direction in Fig. 4) and is scanned in the opposite negative direction (a leftward direction in Fig. 4). Next, the field of view moves in the Y direction again and is scanned in the positive direction again. By repeating reciprocation of the field of view in the X direction and movement of the field of view in the Y direction, the inside of the cultivation container 20 is two-dimensionally scanned.

In the present embodiment, as illustrated in Fig. 5 and Fig. 6, the first displacement sensor 38a and the second displacement sensor 38b are arranged in the X direction with the image forming optical system C interposed therebetween. A field of view R of the image forming optical system C two-dimensionally scans the inside of the cultivation container 20 as described above. At this point, the Z-directional position of the cultivation container 20 is detected at a position that is further in a movement direction of the field of view R than a position of the field of view R of the image forming optical system C with respect to the cultivation container 20. Specifically, in a case where the field of view R is moving in an arrow direction (a rightward direction in Fig. 5) illustrated in Fig. 5, the Z-directional position of the cultivation container 20 is detected by the first displacement sensor 38a that is further in the movement direction of the field of view R between the first displacement sensor 38a and the second displacement sensor 38b. In a case where the field of view R moves from the position illustrated in Fig. 5 to a position of the first displacement sensor 38a, the autofocus control is performed using the previously detected Z-directional positional information of the cultivation container 20, and the partial images are captured.

In a case where the field of view R is moving in an arrow direction (a leftward direction in Fig. 6) in Fig. 6, the Z-directional position of the cultivation container 20 is detected by the second displacement sensor 38b that is further in the movement direction of the field of view R between the first displacement sensor 38a and the second displacement sensor 38b. In a case where the field of view R moves from the position illustrated in Fig. 6 to a position of the second displacement sensor 38b, the autofocus control is performed using the previously detected Z-directional positional information of the cultivation container 20, and the phase difference images are captured.

The detection of the cultivation container 20 using the first displacement sensor 38a and the detection of the cultivation container 20 using the second displacement sensor 38b are switched depending on the movement direction of the field of view R. Accordingly, the Z-directional positional information of the cultivation container 20 at the position of the field of view R can be always acquired before the capturing of the phase difference images in the field of view R.

Based on the Z-directional positional information of the cultivation container 20 detected beforehand as described above, the microscope device control unit 41 performs the autofocus control by controlling driving of the focus adjustment mechanism 35. Specifically, a relationship between the Z-directional positional information of the cultivation container 20 and a movement amount of the image forming optical system C in the optical axis direction is set in advance in the microscope device control unit 41. The microscope device control unit 41 obtains the movement amount of the image forming optical system C in the optical axis direction based on the input Z-directional positional information of the cultivation container 20 and outputs a control signal corresponding to the movement amount to the focus adjustment mechanism 35. The focus adjustment mechanism 35 is driven based on the input control signal. Accordingly, the phase difference lens moves in the optical axis direction, and focus adjustment corresponding to the Z-directional position of the cultivation container 20 is performed.

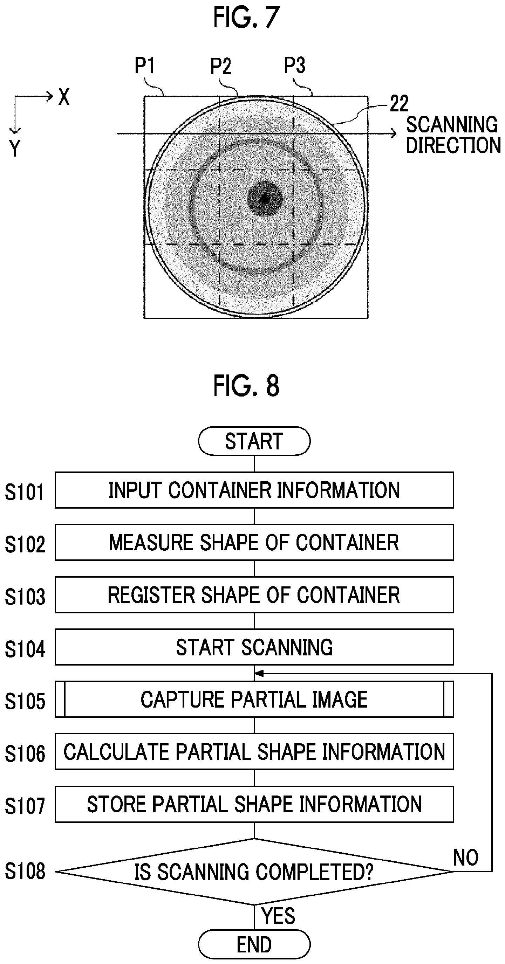

Fig. 7 is a diagram illustrating an example of the partial images acquired by scanning of the microscope device.

In a case where scanning is performed as described thus far, for example, one accommodation part 22 of the cultivation container 20 is covered with nine partial images P divided by dot-dashed lines as illustrated in Fig. 7. As illustrated in Fig. 7, it is ideal that the adjacent partial images P do not deviate and are consistent. However, the partial images may be inconsistent due to acceleration and deceleration in a case where a scanning direction is switched between the main scanning direction and the sub-scanning direction, or vibration of the placing stand 10 or the microscope device 30. The reason for the inconsistency is that imaging is performed at the predetermined imaging positions regardless of the presence or absence of vibration. The observation apparatus of the present embodiment performs the following control so that the adjacent partial images are consistent. An algorithm illustrated below is implemented by causing the CPU to execute the program stored in the storage unit 45.

Fig. 8 is a flowchart illustrating a flow of observation method executed by the observation apparatus. Each step is particularly executed by the control unit 40.

First, the control unit 40 receives an input for the container information related to the cultivation container 20 in the input unit 46 (step S101). For example, the container information of the cultivation container 20 includes specifications (a size, a number, intervals, and the like of accommodation parts 22) of the cultivation container and a model number and a brand of the cultivation container. Information of the number of accommodation parts 22 of the currently used cultivation container 20, the intervals of the accommodation parts 22, a diameter of the accommodation part 22, and the like is obtained from the container information. In addition, since a position and the like of arrangement of the accommodation parts 22 may vary depending on the brand even in a case where the specifications are the same, information of the brand is also effective for specifying the shape of the cultivation container 20.

Next, the control unit 40 measures the shape of the cultivation container 20 not accommodating the observation target in the accommodation part 22 by the measurement unit (step S102). As described above, the measurement unit may not be included in the configuration of the observation apparatus and may be an external device or the microscope device 30. In a case where the shape of the cultivation container 20 is measured, the container information acquired in step S101 is used. For example, the cultivation container 20 is irradiated with laser light by the laser length measurement device, and a solid shape of the container is measured using reflected laser light. At this point, a measurement result of the laser length measurement device can be corrected using the container information. Accordingly, the measurement result of the solid shape of the cultivation container 20 can be more accurate.

The control unit 40 stores shape information that represents the shape of the cultivation container 20 and is obtained in step S102 in the storage unit 45 as the reference shape information (step S103). Step S101 to step S103 are preprocesses for observation performed by the observation apparatus. Accordingly, step S101 to step S103 may be executed in advance regardless of step S104 and subsequent steps.

The control unit 40 starts scanning by starting imaging performed by the microscope device 30 while moving the placing stand 10 by the scanning control unit 42 (step S104).

The control unit 40 captures the partial images of the cultivation container 20 and the observation target by scanning of the microscope device 30 (step S105). Generally, the partial images are captured in a case where the image forming optical system C of the microscope device 30 reaches the predetermined imaging positions. However, the adjacent partial images may be inconsistent due to vibration, acceleration and deceleration, or the like. In such a case, the control unit 40 changes the imaging position of the subsequently captured partial image by controlling the imaging unit 37 and the movement unit 12. Details of a process of capturing the partial images will be described later.

The control unit 40 as a calculation unit calculates the partial shape information representing the partial shape of the cultivation container 20 from the partial images captured in step S105 (step S106). In the present embodiment, the calculated partial shape information is information that indicates the edge of the circular shape of the accommodation part 22 of the cultivation container 20. There is a high likelihood that a part of the edge of the circular shape is included in the partial image. The shape of the edge is effective for checking the consistency between the adjacent partial images. For example, as illustrated in Fig. 7, in a case where nine partial images are obtained, the partial images other than the partial image at the center include a part of the circular shape of the accommodation part 22. In step S106, the partial shape information indicating the shape and the position of the edge of the accommodation part 22 is calculated as a feature included in the partial images.

The control unit 40 stores the calculated partial shape information in the storage unit 45 (step S107).

The control unit 40 decides whether or not the scanning is completed by determining whether or not the imaging is finished at all imaging positions by the microscope device 30 (step S108). In a case where the scanning is not completed (step S108: NO), the process from step S105 is repeated. In a case where the scanning is completed (step S108: YES), the control unit 40 finishes the process of the observation method.

Next, the process of capturing the partial images in step S105 will be described in detail.

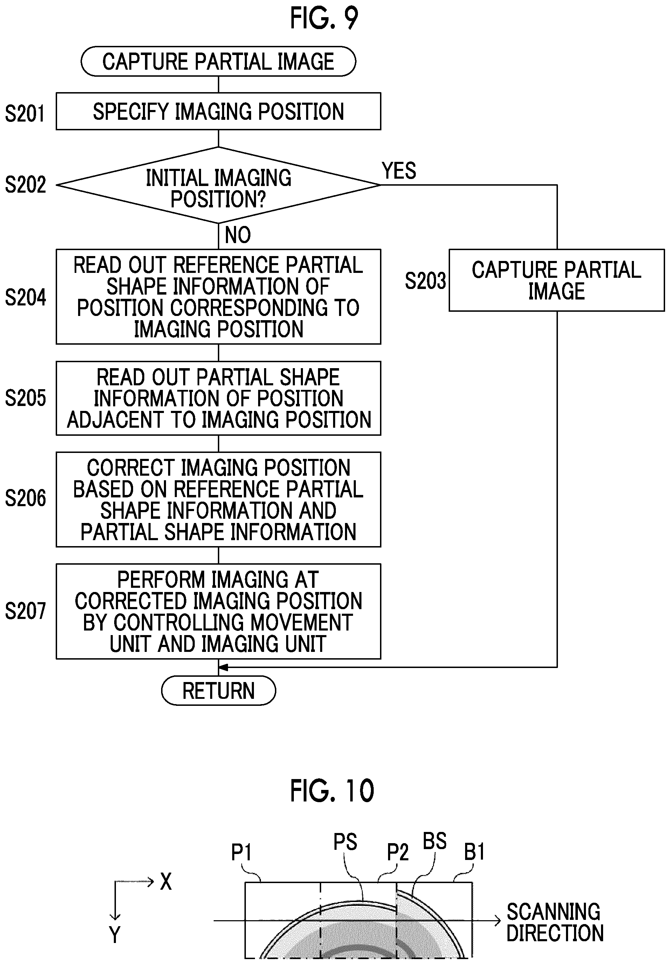

Fig. 9 is a flowchart illustrating a detailed flow of capturing of the partial images.

The control unit 40 specifies the subsequent imaging position (step S201). The imaging positions are stored in advance in the storage unit 45 and are sequentially specified along with a progress of the scanning.

The control unit 40 decides whether or not the subsequent imaging position specified in step S201 is the initial imaging position in the series of imaging positions (step S202). In a case where the subsequent imaging position is the initial imaging position (step S202: YES), the partial images are captured at the predetermined imaging positions (step S203), and a return is made to the flowchart in Fig. 8. In a case where the subsequent imaging position is not the initial imaging position (step S202: NO), the control unit 40 transitions to a process of step S204.

The control unit 40 reads out the reference partial shape information of a position corresponding to the subsequent imaging position from the storage unit 45 (step S204). Alternatively, the control unit 40 may calculate the reference partial shape information corresponding to the subsequent imaging position from the reference shape information based on the reference shape information stored in the storage unit 45 and the subsequent imaging position.

The control unit 40 reads out the partial shape information of the captured partial image adjacent to the subsequent imaging position specified in step S201 from the storage unit 45 (step S205). Accordingly, the partial shape of the captured partial image and the reference partial shape information corresponding to the imaging position that is adjacent to the partial shape and is subsequently imaged are read out.

Based on the read partial shape information and reference partial shape information, the control unit 40 calculates a positional deviation between the imaging position of the captured partial image and the subsequent imaging position and corrects the subsequent imaging position (step S206). Specifically, in a boundary part between the captured partial image and the imaging field of view of the subsequent predetermined imaging position, the control unit 40 calculates a degree to which the position of the edge of the accommodation part 22 deviates in the imaging field of view of the subsequent imaging position from the position of the edge of the accommodation part 22 in the captured partial image. The boundary part includes an adjacent side of the adjacent partial images or an adjacent side between the partial image and the imaging field of view at the subsequent imaging position. For example, the boundary part may be a region that extends inwards at a predetermined ratio from the adjacent side, or may be boundary lines of the adjacent partial images. The control unit 40 decides a direction in which correction for shifting the subsequent imaging position is to be performed such that the position of the partial shape of the captured partial image is arranged in consistency with the position of the reference partial shape in the imaging field of view of the subsequent imaging position in the boundary part. It is preferable that the control unit 40 corrects the imaging position to completely remove the deviation. However, in a case where correcting the imaging position to completely remove the deviation needs to stop or excessively drive the movement unit 12 or causes an unreasonable imaging timing of the imaging unit 37, it may not be necessary to completely remove the deviation. In this case, the control unit 40 corrects the imaging position in a direction in which the deviation is reduced as far as possible.

The control unit 40 images the cultivation container 20 and the observation target at the imaging position corrected in step S206 by controlling the movement unit 12 and the imaging unit 37 (step S207). That is, at least one of the scanning direction or the timing of the imaging performed by the imaging unit 37 is adjusted such that the imaging unit 37 can perform the imaging at the corrected imaging position. In a case where the reference partial shape of the subsequent imaging position does not deviate from the partial shape of the partial image, the control unit 40 does not adjust the scanning direction and the timing of the imaging.

A specific example will be described with reference to Fig. 10.

Fig. 10 is a diagram illustrating a deviation between the partial shape and the reference partial shape.

In the example illustrated in Fig. 10, a state where a series of partial images is obtained in order from the left to the right by the scanning is illustrated. In Fig. 10, a partial image P1 on the left and a partial image P2 at the center show the captured partial images. A partial image B 1 on the right shows a partial image that is obtained in a case where it is assumed that the reference shape of the container is imaged at the subsequent imaging position.

Since capturing is performed up to the partial image P2, the reference partial shape information that indicates a reference partial shape BS included in the partial image B1 corresponding to the imaging position subsequent to the partial image P2 is read out in step S204. Then, in step S205, the partial shape information that indicates a partial shape PS included in the partial image P2 adjacent to the partial image B1 is read out. As illustrated in Fig. 10, in a case where the reference partial shape BS deviates from the calculated partial shape PS in the -Y direction and the imaging is performed at the imaging position, a partial image including a partial shape of which the position deviates in the same manner is captured. That is, the position of the partial shape deviates in the boundary part between the partial images. Thus, the adjacent partial images are inconsistent. In order to capture the subsequent partial image that is consistent with the partial image P2, the imaging position may be corrected in the Y direction in accordance with the deviation of the reference partial shape BS from the partial shape PS. In step S206, the control unit 40 corrects the imaging position and controls the movement unit 12 and the imaging unit 37 such that the imaging unit 37 reaches the corrected imaging position.

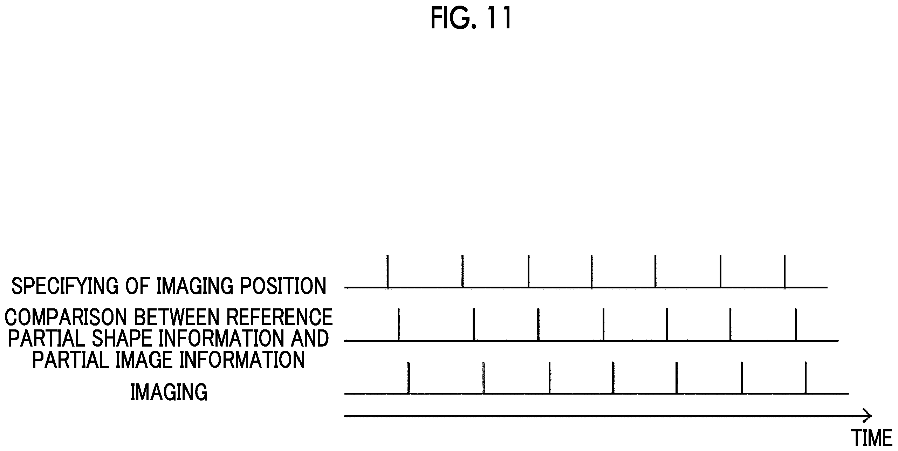

Fig. 11 is a diagram illustrating a timing of operation performed by the control unit 40.

As illustrated in Fig. 11, specifying of the imaging position, comparison between the reference partial shape and the partial shape, and the imaging performed by the imaging unit 37 are sequentially repeated. The imaging position is specified between imaging timings, and the imaging position is corrected based on the comparison between the reference partial shape and the partial shape. That is, the control unit 40 causes the imaging unit to perform imaging at the imaging positions that are sequentially determined such that the adjacent partial images are consistent, based on the reference partial shape of the container prepared in advance and the partial shape calculated in real time by the calculation unit 44. Since the imaging position of the imaging unit 37 is appropriately decided during a series of imaging and the movement unit 12 and the imaging unit 37 are controlled, re-imaging after the end of the series of imaging or re-imaging of the same partial image by suspending the series of imaging does not occur. Consequently, re-imaging is not necessary, and consistent adjacent partial images can be acquired. A favorable entire image of the cell or the like can be acquired by combining the consistent partial images.

In a case where the shape of the cultivation container 20 is measured in advance, the container information is input in advance. Accordingly, the cultivation container 20 can be measured using the container information, and the shape of the cultivation container 20 is obtained with higher accuracy. However, the container information may not be necessarily input. Even in a case where the container information is not present, the cultivation container 20 can be measured in advance based on various inputs provided by the user or information of the previously used imaging positions.

While one embodiment of the present disclosure is described thus far, the technical idea of the present disclosure is not limited to the embodiment. Various modifications can be made.

In the embodiment, the reference partial shape information corresponding to the series of imaging positions is calculated from the reference shape including the shape of the entire cultivation container 20 and is compared with the partial shape information indicating the captured adjacent partial shape as the imaging position. However, instead of comparing the adjacent reference partial shape information and partial shape information as the imaging position, the reference partial shape information and the partial shape information corresponding to the same location as the imaging position may be compared. Even in this case, a deviation amount of the position of the reference partial shape from the position of the partial shape can be determined, and the subsequent imaging position can be corrected in accordance with the deviation amount. In addition, the reference shape information and the partial shape information can be directly compared without using the reference partial shape information. In this case, for example, a position to which the partial shape corresponds in the reference shape indicated by the reference shape information is specified, and a positional deviation between the partial shape and the reference shape can be determined by comparing the specified position with the imaging position at which the partial shape is actually imaged. The subsequent imaging position can be corrected based on the determined deviation.

In the embodiment, the partial shape information and the reference partial shape information (reference shape information) are calculated using the edge of the accommodation part 22 of the cultivation container 20 as a feature. However, instead of the edge of the accommodation part 22, an edge of the entire cultivation container 20 can be calculated as a feature. Alternatively, a mark having a specific shape such as a protruding portion or a recessed portion can be disposed in the cultivation container 20, and a shape of the mark can be calculated as a feature.

In the embodiment, the imaging position is corrected based on the comparison between the reference partial shape and the partial shape. However, considering one accommodation part 22 illustrated in Fig. 7, the partial shape of the cultivation container 20 is not included in the image positioned at the center. Accordingly, in the image positioned at the center in Fig. 7, since the partial shape to be compared is not present, the imaging position cannot be corrected based on a comparison result. Before the image at the center is captured, capturing of the partial image P2 on an upper side and the partial image on a right side from a viewpoint of the image at the center are completed. A correction amount of the imaging position is known for the partial image of which the capturing is completed. Accordingly, considering the known correction amount, the imaging position of the image at the center may be corrected such that a gap is decreased with respect to at least the captured image. That is, in a case where imaging is performed at the imaging position at which the partial shape of the cultivation container 20 is not included in the field of view, a deviation of the imaging position from the original imaging position due to correction is decided from the correction amount of the imaging position in a case where the captured image is captured. In a case where the imaging position of the image at the center is corrected in accordance with the deviation, the imaging position is set in accordance with at least the correction performed up to the present, and a significant deviation of only the image at the center from other images does not occur. In a case where the correction amount in the X direction and the Y direction is different between the captured imaging positions, the imaging position of the image at the center may be corrected using an average value. In the example in Fig. 7, only the image at the center does not include the partial shape but is merely for illustrative purposes. In a case where an image that does not include the partial shape and is not at the center is captured, the current imaging position can be corrected considering the correction amounts of other captured imaging positions as described above.

An observation process that is executed by causing the CPU to read software (program) in the embodiment may be executed by various processors other than the CPU. In this case, the processors are illustrated by a programmable logic device (PLD) such as a field-programmable gate array (FPGA) of which a circuit configuration can be changed after manufacturing, a dedicated electric circuit such as an application specific integrated circuit (ASIC) that is a processor having a circuit configuration dedicatedly designed to execute a specific process, and the like. In addition, the observation process may be executed by one of the various processors or may be executed by a combination of two or more processors of the same type or different types (for example, a plurality of FPGAs and a combination of a CPU and an FPGA). In addition, a hardware structure of the various processors is more specifically an electric circuit in which circuit elements such as semiconductor elements are combined.

In the embodiment, an aspect in which the program of the observation process is stored (installed) in advance in the storage unit 45 is described. However, the aspect is not for limitation purposes. The program may be provided in a form of a recording on a recording medium such as a compact disk read only memory (CD-ROM), a digital versatile disk read only memory (DVD-ROM), and a Universal Serial Bus (USB) memory. In addition, the program may be downloaded from an external device through a network.

### Explanation of References

10: placing stand
11: movement unit
20: cultivation container
21: plate
22: accommodation part
30: microscope device
31: light source
32: slit
33: condenser lens
34: objective lens
35: focus adjustment mechanism
36: image forming lens
37: imaging unit
38: autofocus detection unit
38a: first displacement sensor
38b: second displacement sensor
40: control unit
41: microscope device control unit
42: scanning control unit
43: display control unit
44: calculation unit
45: storage unit
46: input unit
47: display unit
B1: partial image
BS: reference partial shape
C: image forming optical system
E: scanning end point
L: illumination light
M: scanning path
P, P1, P2: partial image
PS: partial shape
R: field of view
S: scanning start point

## Claims

1. An observation apparatus comprising:
an imaging unit (37) adapted to image a container (20) accommodating an observation target in an accommodation part (22) and the observation target in a field of view smaller than the accommodation part at predetermined imaging positions and acquires a series of partial images;
a movement unit (11) adapted to relatively move at least one of the container (20) or the imaging unit (37) with respect to the other;
a calculation unit (44) adapted to calculate partial shape information representing a partial shape (PS) of the container (20) from the partial image obtained by imaging performed by the imaging unit (57);
a storage unit (45) adapted to store reference shape information representing a shape of the container; and
a control unit (40) that, in a case where the partial image scheduled to be subsequently captured during acquisition of the series of partial images is captured is adapted to correct the imaging position based on the partial shape information (PS) and the reference shape information, and to cause the series of partial images in which adjacent partial images are consistent to be captured by controlling the movement unit and the imaging unit based on the corrected imaging position.

2. The observation apparatus according to claim 1,
wherein the calculation unit is adapted to calculate reference partial shape information corresponding to an imaging field of view of each partial image from the reference shape information based on the predetermined imaging positions of the series of partial images, and
based on the partial shape information in one partial image of the adjacent partial images and the reference partial shape information corresponding to the imaging field of view of the other partial image scheduled to be captured subsequently to the one partial image, the control unit (40) is adapted to cause the other partial image to be consistent with the one partial image by correcting the imaging position of the other partial image from the predetermined imaging positions.

3. The observation apparatus according to claim 2,
wherein the control unit (40) is adapted to reduce a deviation between a position of a partial shape of the accommodation part (22) included in the one partial image and a position of a partial shape of the accommodation part (22) included in the other partial image in a boundary part between the adjacent partial images by correcting the imaging position of the other partial image from the predetermined imaging positions.

4. The observation apparatus according to any one of claims 1 to 3,
wherein the storage unit (45) is adapted to store shape information representing a shape of an edge of the accommodation part included in the container as the reference shape information, and
the calculation unit (44) is adapted to calculate shape information representing a shape of an edge of the accommodation part included in the partial image as the partial shape information.

5. The observation apparatus according to any one of claims 1 to 4, further comprising:
an input unit (46) into which the reference shape information can be input,
wherein the control unit (40) is adapted to decide the predetermined imaging positions based on the reference shape information input into the input unit (46).

6. The observation apparatus according to any one of claims 1 to 4, further comprising:
a measurement unit that acquires the reference shape information by measuring the shape of the container,
wherein the control unit (40) is adapted to decide the predetermined imaging positions based on the reference shape information obtained by measurement performed by the measurement unit.

7. An observation method comprising:
an imaging step of imaging a container accommodating an observation target in an accommodation part and the observation target in a field of view smaller than the accommodation part at predetermined imaging positions and acquiring a series of partial images;
a calculation step of calculating partial shape information representing a partial shape of the container from the partial image obtained by imaging performed in the imaging step;
a storage step of storing reference shape information representing a shape of the container; and
a control step of correcting the imaging position in a case where the partial image scheduled to be subsequently captured during acquisition of the series of partial images is captured, based on the partial shape information and the reference shape information, and causing the series of partial images in which adjacent partial images are consistent to be captured in the imaging step based on the corrected imaging position.

8. A non-transitory computer readable recording medium storing an observation program for an observation apparatus comprising
an imaging unit (37) adapted to image a container (20) accommodating an observation target in an accommodation part (22) and the observation target in a field of view smaller than the accommodation part at predetermined imaging positions and acquires a series of partial images;
a movement unit (11) adapted to relatively move at least one of the container (20) or the imaging unit (37) with respect to the other, the program causing a computer to execute:
a calculation step of calculating partial shape information representing a partial shape of the container from the partial image obtained by imaging performed in the imaging step;
a storage step of storing reference shape information representing a shape of the container; and
a control step of correcting the imaging position in a case where the partial image scheduled to be subsequently captured during acquisition of the series of partial images is captured based on the partial shape information and the reference shape information, and causing the series of partial images in which adjacent partial images are consistent to be captured in the imaging step based on the corrected imaging position.

## Patentansprüche

1. Beobachtungsvorrichtung, umfassend:
eine Abbildungseinheit (37), ausgebildet zum Abbilden eines Behälters (20), der ein Beobachtungsziel in einem Aufnahmeteil (22) aufnimmt, und des Beobachtungsziels in einem Gesichtsfeld kleiner als der Aufnahmeteil an vorbestimmten Abbildungspositionen, und zum Erfassen einer Reihe von Teilbildern;
eine Bewegungseinheit (11), ausgebildet zum relativen Bewegen des Behälters in (20) und/oder der Abbildungseinheit (37) in Bezug auf einander;
eine Berechnungseinheit (44), ausgebildet zum Berechnen von Teilforminformation, die eine Teilform (PS) des Behälters (20) repräsentiert, aus dem Teilbild, das durch seitens der Abbildungseinheit (57) ausgeführte Abbildung gewonnen wird;
eine Speichereinheit (45), ausgebildet zum Speichern von Referenz-Forminformation, die eine Form des Behälters repräsentiert; und
eine Steuereinheit (40), die dann, wenn das planmäßig anschließend während der Erfassung der Folge von Teilbildern aufgenommene Teilbild aufgenommen wird, ausgebildet ist zum Korrigieren der Abbildungsposition basierend auf der Teilbildinformation (PS) und der Referenz-Forminformation, und zum Veranlassen, dass die Folge von Teilbildern, in denen benachbarte Teilbilder konsistent sind, aufgenommen wird durch Steuern der Bewegungseinheit und der Abbildungseinheit basierend auf der korrigierten Abbildungsposition.

2. Beobachtungsvorrichtung nach Anspruch 1,
bei der die Berechnungseinheit ausgebildet ist zum Berechnen von Referenz-Teilbildinformation entsprechend einem Abbildungs-Gesichtsfeld jedes Teilbilds aus der Referenz-Forminformation, basierend auf den vorbestimmten Abbildungspositionen der Folge von Teilbildern, und
basierend auf der Teil-Forminformation in einem Teilbild der benachbarten Teilbilder und der Referenz-Teilbildinformation entsprechend dem Abbildungs-Gesichtsfeld des anderen Teilbilds, welches planmäßig im Anschluss an das eine Teilbild aufzunehmen ist, die Steuereinheit (40) ausgebildet ist, um zu veranlassen, dass das andere Teilbild mit dem einen Teilbild konsistent ist, wozu die Abbildungsposition des anderen Teilbilds aus den vorbestimmten Abbildungspositionen korrigiert wird.

3. Beobachtungsvorrichtung nach Anspruch 2,
bei der die Steuereinheit (40) ausgebildet ist zum Reduzieren einer Abweichung einer Position einer Teilform des Aufnahmeteils (22) in dem einen Teilbild und einer Position einer Teilform des Aufnahmeteils (22) in dem anderen Teilbild innerhalb eines Begrenzungsteils zwischen den benachbarten Teilbildern, indem die Abbildungsposition des anderen Teilbilds aus den vorbestimmten Abbildungspositionen korrigiert wird.

4. Beobachtungsvorrichtung nach einem der Ansprüche 1 bis 3,
bei der die Speichereinheit (45) ausgebildet ist zum Speichern von Forminformation, die eine Form eines Rands des Aufnahmeteils repräsentiert, der in dem Behälter enthalten ist, als die Referenz-Forminformation, und
die Berechnungseinheit (44) ausgebildet ist zum Berechnen von Forminformation, die eine Form eines Rands des Aufnahmeteils in dem Teilbild als die Teil-Forminformation zu berechnen.

5. Beobachtungsvorrichtung nach einem der Ansprüche 1 bis 4, weiterhin umfassend:
eine Eingabeeinheit (46), in die die Referenz-Forminformation eingebbar ist,
wobei die Steuereinheit (40) ausgebildet ist zum Entscheiden der vorbestimmten Abbildungspositionen basierend auf der in die Eingabeeinheit (46) eingegebenen Referenz-Form information.

6. Beobachtungsvorrichtung nach einem der Ansprüche 1 bis 4, weiterhin umfassend:
eine Messeinheit, die die Referenz-Forminformation durch Messen der Form des Behälters erfasst,
wobei die Steuereinheit (40) ausgebildet ist zum Entscheiden der vorbestimmten Abbildungspositionen basierend auf der Referenz-Forminformation, die durch Messen seitens der Messeinheit gewonnen wurde.

7. Beobachtungsverfahren, umfassend:
einen Abbildungsschritt des Abbildens eines ein Beobachtungsziel in einem Aufnahmeteil aufnehmenden Behälters und des Beobachtungsziels in einem Gesichtsfeld kleiner als der Beobachtungsteil an vorbestimmten Abbildungspositionen, und Erfassen einer Folge von Teilbildern;
einen Berechnungsschritt des Berechnens von Teil-Forminformation, die eine Teilform des Behälters repräsentiert, aus dem durch in dem Abbildungsschritt ausgeführten Abbilden gewonnen Teilbild;
einen Speicherschritt des Speicherns von Referenz-Forminformation, die eine Form des Behälters repräsentiert; und
einen Steuerschritt des Korrigierens der Abbildungsposition dann, wenn das während der Erfassung der Folge von Teilbildern planmäßig anschließend aufzunehmende Teilbild aufgenommen wird, basierend auf der Teil-Forminformation und der Referenz-Forminformation, und des Veranlassens der Aufnahme der Folge von Teilbildern, in der benachbarte Teilbilder konsistent sind, im Rahmen des Abbildungsschritts basierend auf der korrigierten Abbildungsposition.

8. Nicht-flüchtiges, computer-lesbares Aufzeichnungsmedium, das ein Beobachtungsprogramm für eine Beobachtungsvorrichtung speichert, umfassend:
eine Abbildungseinheit (37), ausgebildet zum Abbilden eines Behälters (20), der ein Beobachtungsziel in einem Aufnahmeteil (22) aufnimmt, und des Beobachtungsziels in einem Gesichtsfeld kleiner als der Aufnahmeteil an vorbestimmten Abbildungspositionen, und zum Erfassen einer Reihe von Teilbildern;
eine Bewegungseinheit (11), ausgebildet zum relativen Bewegen des Behälters in (20) und/oder der Abbildungseinheit (37) in Bezug auf einander;
eine Berechnungseinheit (44), ausgebildet zum Berechnen von Teilforminformation, die eine Teilform (PS) des Behälters (20) repräsentiert, aus dem Teilbild, das durch seitens der Abbildungseinheit (57) ausgeführte Abbildung gewonnen wird;
wobei das Programm einen Computer veranlasst, Folgendes auszuführen:
einen Berechnungsschritt des Berechnens von Teil-Forminformation, die eine Teilform des Behälters repräsentiert, aus dem durch in dem Abbildungsschritt ausgeführten Abbilden gewonnen Teilbild;
einen Speicherschritt des Speicherns von Referenz-Forminformation, die eine Form des Behälters repräsentiert; und
einen Steuerschritt des Korrigierens der Abbildungsposition dann, wenn das während der Erfassung der Folge von Teilbildern planmäßig anschließend aufzunehmende Teilbild aufgenommen wird, basierend auf der Teil-Forminformation und der Referenz-Forminformation, und des Veranlassens der Aufnahme der Folge von Teilbildern, in der benachbarte Teilbilder konsistent sind, im Rahmen des Abbildungsschritts basierend auf der korrigierten Abbildungsposition.

## Revendications

1. Appareil d'observation, comprenant :
une unité d'imagerie (37), apte à imager un récipient (20) logeant une cible d'observation dans une partie de logement (22) et la cible d'observation dans un champ de vision plus petit que la partie de logement sur des positions d'imagerie prédéterminées, et à acquérir une série d'images partielles ;
une unité de déplacement (11), apte à déplacer de manière relative au moins un élément parmi le récipient (20) ou l'unité d'imagerie (37) l'un par rapport à l'autre ;
une unité de calcul (44), apte à calculer des informations de forme partielle représentant une forme partielle (PS) du récipient (20) à partir de l'image partielle obtenue par l'imagerie réalisée par l'unité d'imagerie (57) ;
une unité de stockage (45), apte à stocker des informations de forme de référence représentant une forme du récipient, et
une unité de commande (40), laquelle est apte, dans un cas où l'image partielle planifiée pour être capturée ensuite durant l'acquisition de la série d'images partielles est capturée, à corriger la position d'imagerie sur la base des informations de forme partielle (PS) et des informations de forme de référence, et à faire en sorte de capturer la série d'images partielles, où des images partielles adjacentes sont cohérentes, en commandant l'unité de déplacement et l'unité d'imagerie sur la base de la position d'imagerie corrigée.

2. Appareil d'observation selon la revendication 1,
dans lequel l'unité de calcul est apte à calculer des informations de forme partielle de référence correspondant à un champ de vision d'imagerie de chaque image partielle à partir des informations de forme de référence sur la base des positions d'imagerie prédéterminées de la série d'images partielles, et
sur la base des informations de forme partielle dans une image partielle des images partielles adjacentes et des informations de forme partielle de référence correspondant au champ de vision d'imagerie de l'autre image partielle planifiée pour être capturée suite à la une image partielle, l'unité de commande (40) est apte à faire en sorte que l'autre image partielle soit cohérente avec la une image partielle en corrigeant la position d'imagerie de l'autre image partielle à partir des positions d'imagerie prédéterminées.

3. Appareil d'observation selon la revendication 2,
dans lequel l'unité de commande (40) est apte à réduire un écart entre une position d'une forme partielle de la partie de logement (22) incluse dans la une image partielle et une position d'une forme partielle de la partie de logement (22) incluse dans l'autre image partielle dans une partie limite entre les images partielles adjacentes, en corrigeant la position d'imagerie de l'autre image partielle à partir des positions d'imagerie prédéterminées.

4. Appareil d'observation selon l'une quelconque des revendications 1 à 3,
dans lequel l'unité de stockage (45) est apte à stocker des informations de forme représentant une forme d'un bord de la partie de logement incluse dans le récipient comme informations de forme de référence, et
l'unité de calcul (44) est apte à calculer des informations de forme représentant une forme d'un bord de la partie de logement incluse dans l'image partielle comme informations de forme partielle.

5. Appareil d'observation selon l'une quelconque des revendications 1 à 4, comprenant en outre :
une unité d'entrée (46), où les informations de forme de référence peuvent être entrées, et
dans lequel l'unité de commande (40) est apte à décider des positions d'imagerie prédéterminées sur la base des informations de forme de référence entrées dans l'unité d'entrée (46).

6. Appareil d'observation selon l'une quelconque des revendications 1 à 4, comprenant en outre :
une unité de mesure, laquelle acquiert les informations de forme de référence en mesurant la forme du récipient, et
dans lequel l'unité de commande (40) est apte à décider des positons d'imagerie prédéterminées sur la base des informations de forme de référence obtenues par une mesure réalisée par l'unité de mesure.

7. Procédé d'observation, comprenant les étapes suivantes :
une étape d'imagerie pour imager un récipient logeant une cible d'observation dans une partie de logement et la cible d'observation dans un champ de vision plus petit que la partie de logement sur des positions d'imagerie prédéterminées, et acquérir une série d'images partielles ;
une étape de calcul pour calculer des informations de forme partielle représentant une forme partielle du récipient à partir de l'image partielle obtenue par l'imagerie réalisée lors de l'étape d'imagerie ;
une étape de stockage pour stocker des informations de forme de référence représentant une forme du récipient, et
une étape de commande pour corriger la position d'imagerie, dans un cas où est capturée l'image partielle planifiée pour être capturée ensuite durant l'acquisition de la série d'images partielles, sur la base des informations de forme partielle et des informations de forme de référence, et faire en sorte de capturer la série d'images partielles, où des images partielles adjacentes sont cohérentes, lors de l'étape d'imagerie sur la base de la position d'imagerie corrigée.

8. Support d'enregistrement lisible par ordinateur non transitoire stockant un programme d'observation pour un appareil d'observation, comprenant :
une unité d'imagerie (37), apte à imager un récipient (20) logeant une cible d'observation dans une partie de logement (22) et la cible d'observation dans un champ de vision plus petit que la partie de logement sur des positions d'imagerie prédéterminées, et à acquérir une série d'images partielles ;
une unité de déplacement (11), apte à déplacer de manière relative au moins un élément parmi le récipient (20) ou l'unité d'imagerie (37) l'un par rapport à l'autre, le programme faisant en sorte qu'un ordinateur exécute les étapes suivantes :
une étape de calcul pour calculer des informations de forme partielle représentant une forme partielle du récipient à partir de l'image partielle obtenue par l'imagerie réalisée lors de l'étape d'imagerie ;
une étape de stockage pour stocker des informations de forme de référence représentant une forme du récipient, et
une étape de commande pour corriger la position d'imagerie, dans un cas où l'image partielle planifiée pour être capturée ensuite durant l'acquisition de la série d'images partielles est capturée, sur la base des informations de forme partielle et des informations de forme de référence, et faire en sorte de capturer la série d'images partielles, où des images partielles adjacentes sont cohérentes, lors de l'étape d'imagerie sur la base de la position d'imagerie corrigée.
